# EUROPÄISCHE PATENTANMELDUNG

(11) **EP 4 437 926 A1**
(43) Veröffentlichungstag der Anmeldung: **02.10.2024**
(21) Anmeldenummer: 23165090.4
(22) Anmeldetag: 29.03.2023
(51) Int. Cl.: A61B 1/00, A61B 1/05, A61B 1/313, A61B 1/015, A61B 1/018, A61B 1/06

(54) **ENDOSKOP MIT NICHTAXIALER KAMERAORIENTIERUNG**

(71) Anmelder: Blazejewski Medi-Tech GmbH, 79350 Sexau (DE)
(72) Erfinder: BLAZEJEWSKI, Reinhold, 79261 Gutach (DE)
(74) Vertreter: Rüger Abel Patentanwälte PartGmbB

(57) **Zusammenfassung**

Das erfindungsgemäße Endoskop (10) weist eine distale Endfläche 16 mit zwei Flächenbereichen (17, 18) auf, die miteinander einen stumpfen Winkel einschließen, wobei ein Flächenbereich (17) zu der Längsrichtung (L) geneigt orientiert ist, während der andere Flächenbereich (18) quer zu der Längsrichtung (L) steht. Die Bildaufnahmeeinrichtung 21 weist ein Objektiv mit einem Lichteintrittsfenster (20) auf, das vorzugsweise flächenbündig zu dem Flächenbereich (17) angeordnet ist, wobei die Bildaufnahmeeinrichtung (21) innerhalb einer zylindrischen längs orientierten Kontur angeordnet ist und dennoch eine schräg zu der Längsrichtung orientierte Blickrichtung aufweist. Das zu der Bildaufnahmeeinrichtung (21) gehörige Objektiv (24) weist eine optische Achse mit wenigstens einem Knick auf, sodass sie einen längsorientierten Abschnitt (26) und einen schräg orientierten äußeren Abschnitt (N20) hat. Hingegen ist die Beleuchtungseinrichtung (23 (33)) längs orientiert und weist einen Abstrahlwinkel auf, der größer ist als der Öffnungswinkel (o) des Objektivs (24) der Bildaufnahmeeinrichtung (21).

## Beschreibung

Die Erfindung betrifft ein Endoskop, insbesondere ein Wirbelsäulenendoskop oder Endoskop für andere Zwecke, z.B. für urologische Anwendungen.

Endoskope zur Inspektion von Operationsorten sind aus dem Stand der Technik bekannt. Sie dienen dazu, einem Operateur oder sonstigen Behandler Sicht auf einen inneren Körperbereich eines Patienten zu gewähren.

Die WO 2022/043835 A1 und die DE 10 2020 008 129 A1 offenbaren ein solches Endoskop mit einem stabförmigen Schaft, an dessen distalen Ende zentral eine Kamera angeordnet ist. Die Kamera weist eine Beleuchtungseinrichtung auf, die durch ringförmig um das Kameraobjektiv herum angeordneten Leuchtdioden gebildet ist. Die Leuchtdioden strahlen durch ein ringförmiges Fenster Licht ab, wobei die Abstrahlrichtungen der einzelnen Leuchtdioden entweder parallel zur optischen Achse der Kamera oder von dieser weg gerichtet sind. Die Lichtkegel der einzelnen Leuchtdioden des Leuchtdiodenkranzes überlappen einander, sodass insgesamt ein Abstrahlwinkel der Beleuchtung entsteht, der 180° übersteigen kann. Allerdings weist das zentral in dem Leuchtdiodenkranz angeordnete Kameraobjektiv einen noch größeren Öffnungswinkel auf.

Die DE 10 2014 208 754 A1 offenbart ein Endoskop mit Arbeitskanal mit Bildaufnahmeeinrichtung und Beleuchtungseinrichtung. Die Beleuchtungseinrichtung umfasst mehrere LED-Lichtquellen, die in einem Viereck um das Kameraobjektiv der Bildaufnahmeeinrichtung herum angeordnet sind. Die Blickrichtung des Kameraobjektivs und die Abstrahlrichtungen der LED Beleuchtungen sind identisch und stimmen mit der Längsrichtung des Arbeitskanals überein.

Auch die CH 698 893 B1 offenbart ein Endoskop mit Kamera und Beleuchtung, die gemeinsam an dem distalen Ende des Endoskopschafts angeordnet sind. Weiterer ähnlicher Stand der Technik wird durch die WO 2019/158168 A1, die WO 2021/175387, die DE 10 2020 134 332 A1, die US 11,529,039 B2, die WO 2021/175386 A1, die US 2019/0246027 A1, die EP 2 415 386 A1, die DE 10 2013 201 809 A1, die EP 2 311 366 B1 und die US 8,308,637 B2 gebildet.

Bei den vorgestellten Endoskopen wird prinzipiell davon ausgegangen, dass die Kamera eine optische Achse aufweist, die parallel zu der Längsrichtung des Endoskopschafts orientiert ist.

Aus der oben genannten WO 2022/043835 A1 geht zusätzlich ein Endoskop hervor, bei dem die aus Beleuchtung und Kamera bestehende Einheit in Schrägausrichtung zur Längsachse des Endoskopschafts eingebaut ist, sodass die Blickrichtung der Kamera auf ein aus dem Arbeitskanal herausragendes Instrument gerichtet ist. Bei dieser Anordnung kann es zu erheblichem Schattenwurf durch das Instrument kommen, was trotz großen Öffnungswinkels der Kamera und der Beleuchtungseinrichtung zu Sichtbeeinträchtigungen führen kann.

Davon ausgehend ist es Aufgabe der Erfindung, Endoskop mit guten Sichtverhältnissen zu schaffen.

Diese Aufgabe wird mit dem Endoskop nach Anspruch 1 gelöst:

Das erfindungsgemäße Endoskop weist einen länglichen Schaft auf, der sich entlang einer Längsrichtung erstreckt und sowohl eine am distalen Ende eingebaute Bildaufnahmeeinrichtung sowie eine ebenfalls am distalen Ende eingebaute Beleuchtungseinrichtung aufweist. Der Schaft kann entlang seiner gesamten Länge starr ausgebildet sein. Alternativ kann der Schaft auch an einer oder mehreren Stellen oder insgesamt flexibel ausgebildet sein. Außerdem kann der Schaft ein gezielt durch entsprechende Betätigungsmittel abwinkelbares distales Ende aufweisen.

Die Bildaufnahmeeinrichtung umfasst einen Bildsensor und ein Objektiv. Die Beleuchtungseinrichtung umfasst ein oder mehrere LED-Lichtquellen, vorzugsweise Weißlicht-LEDs. An der distalen Endfläche des Endoskops sind ein erstes Fenster, das der Bildaufnahmeeinrichtung zugeordnet ist, sowie ein zweites Fenster vorgesehen, das der Beleuchtungseinrichtung zugeordnet ist. Das erste Fenster und das zweite Fenster sind auf einander zu geneigt. Dies bedeutet, dass eine Flächennormale des ersten Fensters und eine Flächennormale des zweiten Fensters miteinander einen spitzen Winkel einschließen. Sind die Fenster nicht eben, sondern gewölbt, ist die Flächennormale diesbezüglich in der Mitte des Fensters anzusetzen. Der spitze Winkel ist in proximaler Richtung geöffnet, d.h. der Schnittpunkt der beiden Flächennormalen liegt distal von der distalen Endfläche des Endoskops entfernt. Schneiden sich die beiden Flächennormalen nicht, liegt der Punkt der größten Annäherung der beiden Flächennormalen aneinander distal vor der distalen Endfläche des Endoskops.

Entsprechend der Neigung des ersten Fensters und des zweiten Fensters zueinander schließen auch die optischen Achsen der Bildaufnahmeeinrichtung und der Beleuchtungseinrichtung miteinander einen spitzen Winkel ein. Die Beleuchtungseinrichtung weist vorzugsweise eine optische Achse auf, die parallel zu der Längsrichtung des Endoskops und somit parallel zu einem optional vorhandenen Arbeitskanal ausgerichtet ist. Die optische Achse der Beleuchtungseinrichtung ist die Mittelachse des von der Beleuchtungseinrichtung emittierten Lichtkegels.

Die optische Achse der Bildaufnahmeeinrichtung ist hingegen zu der Längsrichtung des Schafts geneigt. Außerdem ist die Bildaufnahmeeinrichtung gegen die Mittelachse des Schafts in Radialrichtung versetzt angeordnet. Mit dieser Konfiguration lässt sich ein Operationsgebiet und die Tätigkeit eines auf biologisches Gewebe einwirkenden Werkzeugs besonders klar und gut erkennen. Durch die Längsausrichtung der Beleuchtungseinrichtung enthält das abgestrahlte Licht, bezogen auf ein parallel orientiertes Werkzeug, Streiflichtanteile, d.h. einige Lichtstrahlen des abgestrahlten Lichtbündels verlaufen im Wesentlichen parallel zu dem aus dem Arbeitskanal ragenden oder parallel zu dem Endoskop eingesetzten Werkzeugs. Die Streiflichtanteile heben Bildbestandteile hervor und führen zu kontrastreichen Bildern. Die von der Seite auf die Szenerie schauende Kamera überblickt das Operationsgebiet gut und kann insbesondere Axialbewegungen des Werkzeugs gut verfolgen.

Das erste Fenster und das zweite Fenster können jeweils ebene Lichteintrittsflächen aufweisen. Diese können flächenbündig in die distale Endfläche des Endoskops eingelassen sein, wodurch sich das Endoskop besonders gut reinigen und sterilisieren lässt.

Wie erwähnt, kann die Bildaufnahmeeinrichtung eine optische Achse aufweisen, die in einem von Null verschiedenen spitzen Winkel zu der Längsrichtung orientiert ist. Dies betrifft insbesondere die optische Achse in ihrem Verlauf ab dem ersten Fenster (Lichteintrittsfenster). Im Verlauf des Lichtwegs zum Bildsensor kann die optische Achse jedoch auch eine oder mehrere Richtungsänderungen erfahren und innerhalb des Objektivs parallel zu der Längsrichtung orientiert sein. Dieser Abschnitt der optischen Achse ist vorzugsweise seitlich gegen die Mittelachse des Schafts versetzt angeordnet. Dies reduziert den radialen Platzbedarf der Bildaufnahmeeinrichtung gegenüber einer insgesamt schräg gestellten Bildaufnahmeeinrichtung mit durchgehend gerader optischer Achse. Dieses Konzept ermöglicht auch die Verwendung von Objektiven mit erheblicher Ausdehnung in Längsrichtung. Solche Objektive können mehrere fest oder verstellbar angeordnete Linsen oder sonstige optische Elemente, wie Prismen, Spiegel oder dergleichen aufweisen. Es lassen sich hochwertige Objektive nutzen, die eine hohe Bildqualität ermöglichen. Dabei kann das Endoskop besonders schlank ausgebildet werden.

Die optische Achse der Bildaufnahmeeinrichtung ergibt sich, wie schon erwähnt, als Mittelachse eines von der Beleuchtungseinrichtung abgestrahlten Lichtkegels. Dieser Lichtkegel weist einen Beleuchtungs-Öffnungswinkel auf, der vorzugsweise größer ist als der Öffnungswinkel des Objektivs. Damit wird der Operationsort großzügig und großflächig ausgeleuchtet.

Es ist vorteilhaft, wenn die distale Endfläche des Endoskops einen ersten ebenen Flächenabschnitt aufweist, dessen Flächennormale gegen die Längsrichtung geneigt ist, wobei die distale Endfläche einen zweiten ebenen Flächenabschnitt aufweist, dessen Flächennormale parallel zur Längsrichtung orientiert ist. Vorzugsweise stimmen die Neigungen der Flächenabschnitte mit den Neigungen der in ihnen vorgesehenen Fenster überein. Dadurch können die Fensterflächen bündig in die Flächenabschnitte der distalen Endfläche eingesetzt sein. Die Flächenabschnitte können an einer Kante absatzlos ineinander übergehen. Bei einer bevorzugten Ausführungsform ist jedoch zwischen den beiden Flächenbereichen ein (kleiner) Absatz angeordnet, indem der das zweite Fenster aufweisende zweite Flächenbereich, der das erste Fenster enthält, in Proximalrichtung gegen den ersten Flächenbereich in Proximalrichtung versetzt ist. Dadurch kann eine direkte Lichteinstrahlung aus dem zweiten Fenster in das erste Fenster und somit die Bildaufnahmeeinrichtung verhindert werden. Dies gilt insbesondere, wenn das zweite Fenster vollkommen klar ist, d.h. keine relevante Lichtstreuung bewirkend ausgebildet ist.

Im einfachsten Fall enthält ein solches Endoskop keinen zusätzlichen Kanal. Jedoch kann das Endoskop auch mit einem oder mehreren Arbeitskanälen versehen sein, die sich längs durch den Schaft erstrecken. Ein solches Endoskop kann nicht nur zur Betrachtung eines Operationsorts, sondern mittels einer durch den Arbeitskanal zum Operationsort geführten Sonde auch zur Einwirkung auf diesen dienen. Vorzugsweise weist der Arbeitskanal eine Mündung auf, die vollständig von dem zweiten Flächenbereich umschlossen ist. In diesem Fall enthält der zweite Flächenbereich mindestens ein, vorzugsweise zwei Lichtaustrittsfenster (zweites und drittes Fenster). Zwischen den Fenstern der Beleuchtungseinrichtung und dem Fenster der Bildaufnahmeeinrichtung können weitere Kanäle vorgesehen sein, die zum Beispiel zum Zu- oder Abführen von Spülfluid dienen. Die Mündungen dieser Kanäle können die beiden Flächenbereiche überschneidend angeordnet sein.

Vorzugsweise umfasst die Beleuchtungseinrichtung lediglich zwei Lichtquellen mit zwei Lichtaustrittsfenstern, die gemeinsam auf einer quer zur Längsrichtung gedachten Linie angeordnet sind, die vorzugsweise etwa mittig zwischen der Bildaufnahmeeinrichtung und dem Arbeitskanal lokalisiert ist. Dadurch ergeben sich vorteilhafte Beleuchtungs- und Bildaufnahmeverhältnisse mit einer guten Übersicht über den Operationsbereich für den Operateur. Außerdem ergibt sich eine optimale Platzausnutzung, sodass das Endoskop besonders schlank gestaltet werden kann. Außerdem können die beiden Lichtquellen dazu eingerichtet sein, Licht in unterschiedlichen Wellenlängebereichen auszusenden. Zusätzlich oder alternativ können eine, beide oder mehrere Lichtquellen lichtemittierende Elemente, z.B. LEDs, aufweisen, die dazu eingerichtet sein, Licht in unterschiedlichen Wellenlängebereichen auszusenden. Z.B. kann ein Wellenlängenbereich im Bereich sichtbaren Lichts liegen und z.B. Weißlicht sein. Ein anderer Wellenlängenbereich kann im Bereich unsichtbaren Lichts liegen, z.B. infraroten, insbesondere nah-infraroten Lichts oder UV-Licht. Das unsichtbare Licht kann zur Anregung von Fluoreszenzmarkern dienen.

Das zweite und das dritte Fenster der Beleuchtungseinrichtung sind dabei an zwei verschiedenen Seiten der Mündung des Arbeitskanals in unmittelbarer Nachbarschaft zu dem ersten Flächenbereich angeordnet. Von der Bildaufnahmeeinrichtung gesehen, liegen alle Lichtquellen der Beleuchtungseinrichtung querab innerhalb eines Winkels von kleiner als 180° bei Endoskopen mit Arbeitskanal und vorzugsweise in einem Winkel von kleiner als 90° bei Endoskopen ohne Arbeitskanal.

Weitere Einzelheiten vorteilhafter Ausführungsformen des Endoskops ergeben sich aus Ausführungsbeispielen und Ansprüchen. Ausführungsbeispiele der Erfindung sind nachstehend im Zusammenhang mit der Zeichnung beschrieben. In der Zeichnung zeigen:
Figur 1 ein erfindungsgemäßes Endoskop, in schematisierter Perspektivdarstellung,
Figur 2 das distale Ende des Endoskops nach Figur 1, in Perspektivdarstellung,
Figur 3 das distale Ende des Endoskops nach Figur 1 und 2, im Längsschnitt,
Figur 4 eine Stirnansicht der distalen Endfläche des Endoskops nach den Figuren 1 bis 3,
Figur 5 das distale Ende einer abgewandelten Ausführungsform des erfindungsgemäßen Endoskops, in Perspektivdarstellung,
Figur 6 das distale Ende des Endoskops nach Figur 5, in Längsschnittdarstellung, und
Figur 7 eine Ansicht der distalen Endfläche des Endoskops nach den Figuren 5 und 6.

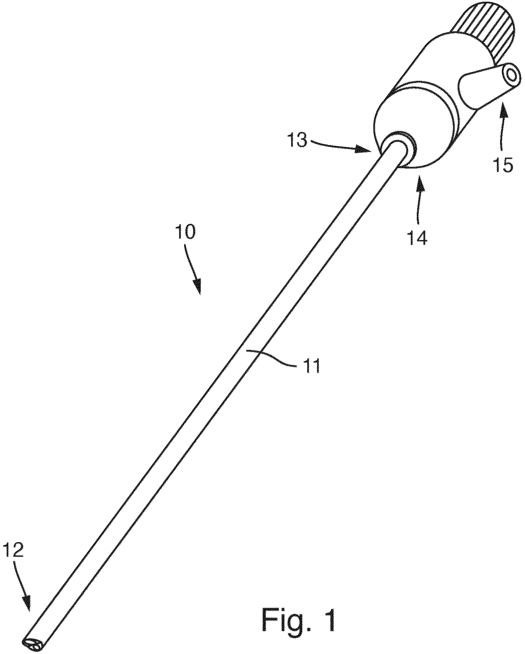

In Figur 1 ist ein Endoskop 10 veranschaulicht, das zur endoskopischen Behandlung von Patienten dient und beispielsweise als Wirbelsäulenendoskop, aber auch als Endoskop für andere Zwecke Anwendung finden kann. Das Endoskop 10 weist einen länglichen Schaft 11 auf, der als starrer Schaft oder in einer anderen Variante auch als flexibler Schaft ausgebildet sein kann. Er erstreckt sich von seinem distalen Ende 12 zu seinem proximalen Ende 13, wo er an einem Endoskopkopf 14 gehalten ist. An dem Endoskopkopf 14 kann ein Anschluss 15 vorgesehen sein, der zum Beispiel zum Anschluss einer Versorgungsleitung, beispielsweise zur Zuführung elektrischer Energie oder auch als Anschluss zum Einführen einer Sonde oder als Anschluss zum Zu - oder Abführen von Fluiden ausgebildet sein kann. Außerdem kann an dem Endoskopkopf ein Anschluss zur Signalübertragung, beispielsweise zur Bildsignalübertragung vorgesehen sein.

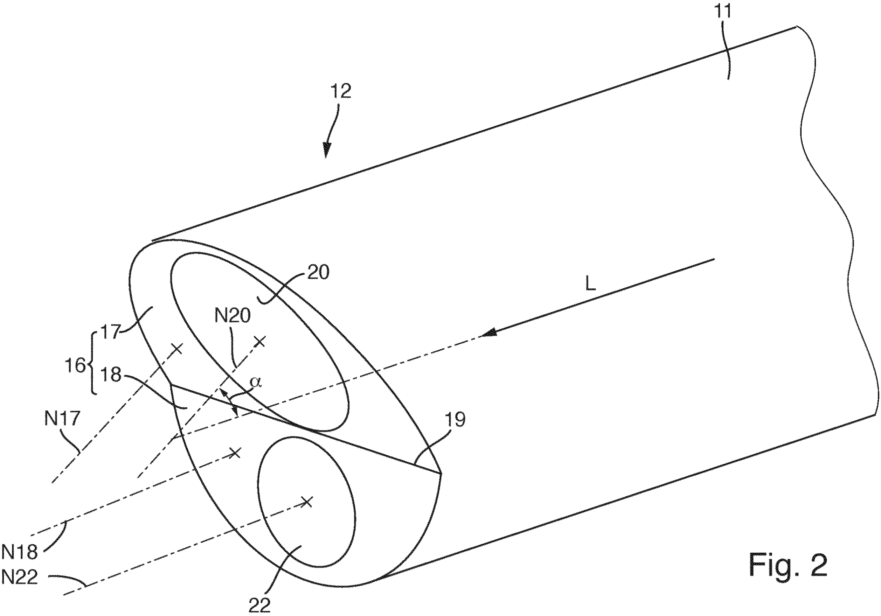

Das distale Ende 12 des Endoskops 10 ist in Figur 2 gesondert veranschaulicht. Der längliche im Querschnitt vorzugsweise kreisrunde Schaft 12 legt mit seiner Längserstreckung eine Längsrichtung L fest, die in Figur 2 durch einen Pfeil symbolisiert ist.

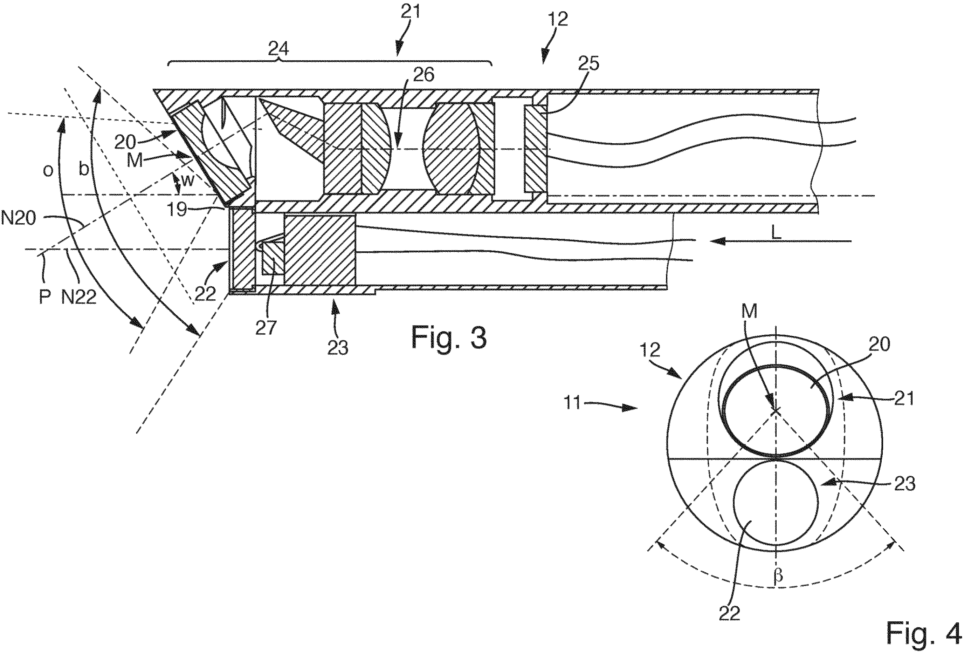

Der längliche zylindrische Schaft 11 weist an seinem distalen Ende 12 eine distale Endfläche 16 auf, zu der ein erster vorzugsweise ebener Flächenbereich 17 und ein zweiter ebenfalls vorzugsweise ebener Flächenbereich 18 gehören, die an einer vorzugsweise geraden Trennlinie 19 ineinander übergehen. Die Flächenbereiche 17, 18 können absatzlos ineinander übergehen. Alternativ kann an der Trennlinie 19 auch eine Stufe vorgesehen sein, wie es aus Figur 3 hervorgeht.

In dem ersten Flächenbereich 17 ist ein erstes Fenster 20 angeordnet, das als Lichteintrittsfenster einer aus Figur 3 ersichtlichen Bildaufnahmeeinrichtung 21 dient. In dem zweiten Flächenbereich 18 ist ein zweites Fenster 22 angeordnet, das als Lichtaustrittsfenster einer Beleuchtungseinrichtung 23 dient. Das erste Fenster 20 und das zweite Fenster 22 können distal eine ebene Fläche aufweisen und flächenbündig mit dem sie jeweils vollständig umgebenden Flächenbereich 17, 18 abschließen.

Der Flächenbereich 17 weist eine Flächennormale N17 auf, die gegen die Längsrichtung L in einem spitzen Winkel w geneigt ist. Von der Trennlinie 19 ausgehend verläuft der Flächenbereich 17 in distaler Richtung ansteigend. Hingegen weist der Flächenbereich 18 eine Flächennormale N18 auf, die parallel zu der Längsrichtung L orientiert ist. Die Flächennormalen N17, N18 schneiden sich somit distal vor der distalen Endfläche 16 in einem Punkt P. Ebenfalls distal vor der distalen Endfläche 16 schneiden sich die Flächennormalen N20, N22 des ersten und zweiten Fensters 20, 22.

Wie Figur 3 veranschaulicht, stimmt die Flächennormale N20 mit der vor dem distalen Ende 12 verlaufenden optischen Achse der Bildaufnahmeeinrichtung 21 überein. Die Bildaufnahmeeinrichtung 21 umfasst ein Objektiv 24 und einen Bildaufnahmesensor 25. Der Strahlengang des Objektivs kann Linsen und weitere optische Elemente, wie Spiegel oder Prismen, enthalten, sodass die optische Achse innerhalb des Objektivs mindestens eine Ablenkung erfährt und dort einen parallel zur Längsrichtung L aufweisenden Abschnitt 26 aufweist. Durch mindestens einen oder auch mehrere Knicke in der optischen Achse gelingt es, das gesamte Objektiv 24 und den Bildaufnahmesensor 25 innerhalb einer in Längsrichtung L orientierten zylindrischen Kontur unterzubringen, obwohl das Objektiv 24 und somit die gesamte Bildaufnahmeeinrichtung 21 eine durch die Flächennormale N20 veranschaulichte, unter einem Winkel α schräg zu der Längsrichtung L verlaufende Blickrichtung aufweist.

Das Objektiv 24 weist vorzugsweise einen Öffnungswinkel o kleiner 120°, vorzugsweise von höchstens 80° oder kleiner auf (in Luft). Unter Wasser kann der Öffnungswinkel o ca. 65° betragen.

Die Beleuchtungseinrichtung 23 weist ein lichtemittierendes Halbleiterelement, wie beispielsweise eine LED 27 auf, die als Weißlicht LED ausgebildet sein kann. Vorzugsweise handelt es sich dabei um ein kurzwelliges Licht ausstrahlende LED mit Leuchtstoff. Alternativ können auch mehrere verschiedenfarbige LED-Chips vorgesehen sein, deren Licht sich zu weißem Licht oder Licht steuerbarer Farbigkeit überlagert. Das von der LED 27 abgestrahlte Licht durchstrahlt das zweite Fenster 22, das vorzugsweise vollkommen durchsichtig oder auch transluzent ausgebildet ist. Der Lichtaustrittswinkel, der den Öffnungswinkel b eines aus dem Fenster 22 austretenden Lichtkegels markiert, ist vorzugsweise größer als der Öffnungswinkel o des Objektivs 24. Der Abstrahl- oder Lichtaustrittswinkel kann beispielsweise zwischen 90° und 120° liegen. Die Mittelachse des von der Beleuchtungseinrichtung 23 ausgestrahlten Lichtkegels wird durch die Flächennormale N22 markiert, die parallel zu der Längsrichtung L orientiert ist.

Wie Figur 4 veranschaulicht, ist die Beleuchtungseinrichtung 23 an lediglich einer Seite der Bildaufnahmeeinrichtung 21 angeordnet. Von einem Mittelpunkt M des ersten Fensters 20 aus gesehen, liegt die Beleuchtungseinrichtung 23 sowie deren Lichtaustrittsfenster 22 innerhalb eines in Figur 4 durch gestrichelte Linien markierten Winkels β, der vorzugsweise kleiner als 90° ist. Durch diese Anordnung kann ein besonders schlanker Schaft 11 gestaltet werden. Dieser kann, wie Figur 4 veranschaulicht, einen Kreisquerschnitt aufweisen. Alternativ, kann der Schaft 11, wie durch gestrichelte Linien angedeutet ist, auch oval ausgebildet sein.

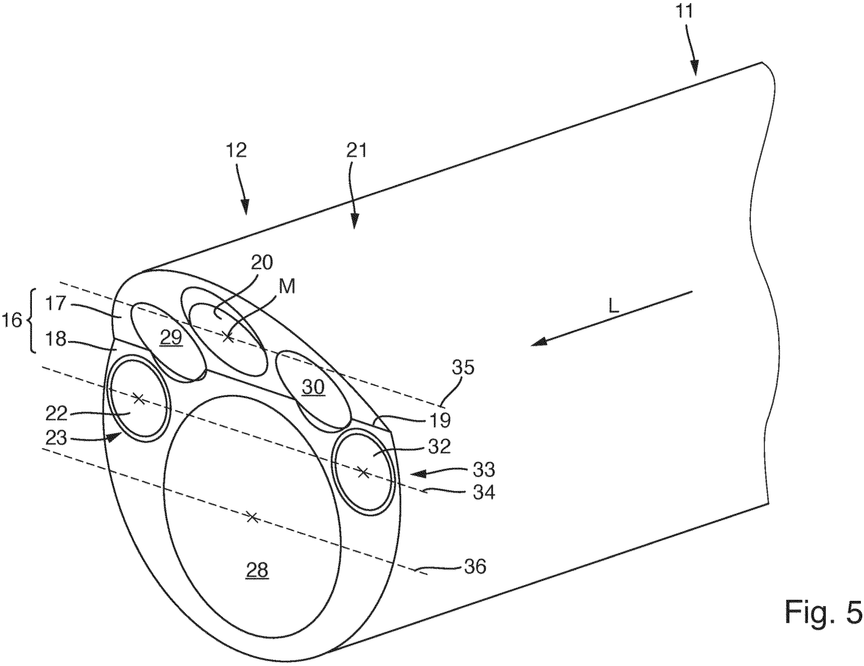

Während die Figuren 1 bis 4 ein Endoskop ohne Arbeitskanal veranschaulichen, ist in den Figuren 5 bis 7 ein Endoskop mit einem Arbeitskanal 28 sowie optional weiteren Kanälen, beispielsweise Spülkanälen 29, 30, veranschaulicht. Der Arbeitskanal 28 wie auch die Spülkanäle 29, 30 erstrecken sich vorzugsweise in Längsrichtung l durch den gesamten Schaft 11 von seinem distalen Ende 12 zu seinem proximalen Ende 13 und dem Endoskopkopf 14 hin, wo sie mit entsprechenden Anschlüssen verbunden sind.

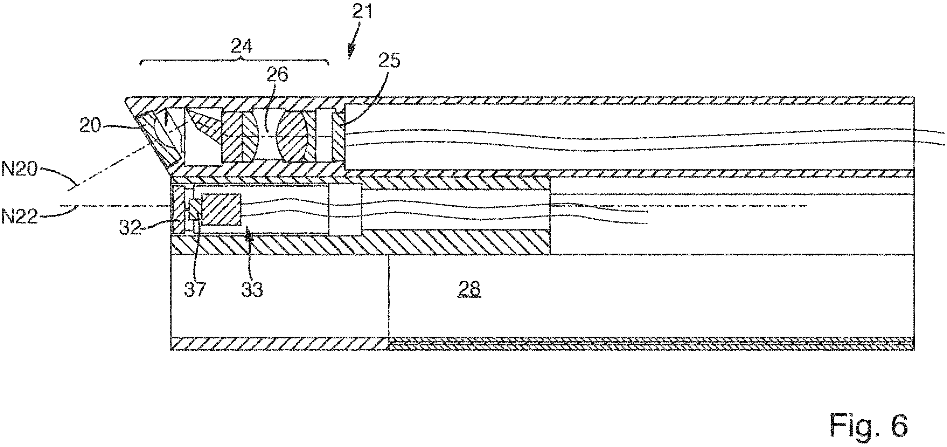

Das Endoskop nach Figur 5 enthält, wie schon zuvor beschrieben, wiederum eine Bildaufnahmeeinrichtung 21, deren Lichteintrittsfenster das erste Fenster 20 ist. Die im Zusammenhang mit den Figuren 1 bis 4 gegebene Beschreibung des Endoskops 10, insbesondere der Bildaufnahmeeinrichtung 21, deren Objektiv 24 und Bildsensor 25 sowie des Schafts 11 und der distalen Endfläche 16, gilt für die in Figur 6 veranschaulichte Bildaufnahmeeinrichtung 21 unter Zugrundelegung der bereits eingeführten Bezugszeichen entsprechend. Die distale Endfläche 16 ist wiederum in Flächenbereiche 17, 18 unterteilt, die, wie im Zusammenhang mit Figur 1 bis 4 beschrieben, zu der Längsrichtung L orientiert sind und an der Stufe oder Trennlinie 19 aneinander grenzen.

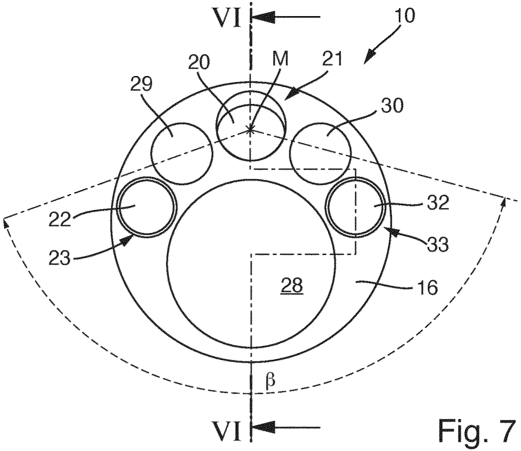

Wie die Figuren 5 und 7 erkennen lassen, ist jedoch zusätzlich zu dem zweiten Fenster 22 ein drittes Fenster 32 vorhanden, das als Lichtaustrittsfenster dient und zu einer weiteren Beleuchtungseinrichtung 33 gehört. Die Beleuchtungseinrichtung 33 ist identisch zu der Beleuchtungseinrichtung 23 ausgebildet. Die Beleuchtungseinrichtungen 23, 33 sind symmetrisch zu einer Ebene angeordnet, die in Längsrichtung L verläuft und mittig durch den Arbeitskanal 28 und das Lichteintrittsfenster 20 geht. Wie Figur 5 zeigt, liegen die Lichteintrittsfenster 22, 32 dabei mit ihren Mittelpunkten auf einer Linie 34, die zwischen Parallelen 35, 36 angeordnet sind, die parallel zu der Trennlinie 19 angeordnet sind, wobei die Parallele 35 mittig durch das erste Fenster 20 und die Parallele 36 mittig durch den Arbeitskanal 28 verläuft. Vorzugsweise ist die Linie 34 etwa mittig zwischen den Linien 35, 36. Der Spülkanal 29 ist vorzugsweise zwischen dem ersten Fenster 20 und dem zweiten Fenster 22 angeordnet. Die Mündung des Spülkanals 30 ist vorzugsweise zwischen dem ersten Fenster 20 und dem dritten Fenster 32 angeordnet. Es ergibt sich so eine optimale Platzausnutzung in dem Schaft 11. Umgekehrt lassen sich dadurch besonders schlanke Endoskope gestalten.

Der mit der Beleuchtungseinrichtung 23 übereinstimmende Aufbau der Beleuchtungseinrichtung 33 ergibt sich aus Figur 6, die eine Schnittdarstellung gemäß der Schnittlinie VI-VI aus Figur 7 ist. Die Beschreibung der Beleuchtungseinrichtung 23 gilt entsprechend für die Beleuchtungseinrichtung 33 und deren LED 37. Wie aus Figur 7 ersichtlich, liegen alle Beleuchtungseinrichtungen 23, 33 des Endoskops 10 nach Figur 7 innerhalb eines Winkels β, dessen Scheitel durch den Mittelpunkt M des ersten Fensters 20 markiert ist, wobei diese Winkel β kleiner als 180° ist.

Das insoweit beschriebene Endoskop 10 arbeitet wie folgt:
Zum Einsatz am Patienten wird das Endoskop 10 durch eine geeignete Öffnung des Körpers des Patienten in einen optisch zu inspizierenden Bereich desselben eingeführt. Im Falle des Endoskops nach den Figuren 1 bis 4 wird die Beleuchtungseinrichtung 23 aktiviert. Das von dem Bildsensor 25 aufgenommene Bild wird in Form von Bildsignalen an einen geeigneten Monitor übertragen. Dieser Monitor kann ein am Endoskopkopf 15 vorgesehener Bildschirm, ein davon abgesetzter gesonderter Bildschirm oder auch eine VR-Brille des Behandlers sein. Wird ein auf anderem Wege in den Sichtbereich der Bildaufnahmeeinrichtung 21 gebrachte Instrument genutzt, kann dieses ebenfalls gesehen und dessen Einsatz überwacht und gesteuert werden. Dabei eignet sich das Endoskop 10 auch für Instrumente, die parallel zu dem Schaft desselben in den Patienten eingeführt und benutzt werden. Durch die spezielle Anordnung der Beleuchtung in Längsrichtung L orientierten Beleuchtungseinrichtung mit der zu der Längsrichtung L hin geneigten Blickrichtung der Bildaufnahmeeinrichtung 21 wird eine gute Übersicht über das Operationsgebiet und die Arbeit des Instruments sichergestellt.

Dies gilt insbesondere für das Endoskop nach den Figuren 5 bis 7, bei dem die Arbeitsrichtung einer durch den Arbeitskanal 28 eingeführten Sonde durch den Arbeitskanal 28 parallel zu der Längsrichtung L vorgegeben ist. Durch die Anordnung zweier Beleuchtungseinrichtungen 23, 33 zu beiden Seiten des Arbeitskanals 28, jedoch leicht zu der Bildaufnahmeeinrichtung 21 hin versetzt, wird eine gute Ausleuchtung des Operationsgebiets ohne Schattenwurf und mit für die Arbeit des Instruments optimierter Blickrichtung erreicht.

Das erfindungsgemäße Endoskop 10 weist eine distale Endfläche 16 mit zwei Flächenbereichen 17, 18 auf, die miteinander einen stumpfen Winkel einschließen, wobei ein Flächenbereich 17 zu der Längsrichtung L geneigt orientiert ist, während der andere Flächenbereich 18 quer zu der Längsrichtung L steht. Die Bildaufnahmeeinrichtung 21 weist ein Objektiv mit einem Lichteintrittsfenster 20 auf, das vorzugsweise flächenbündig zu dem Flächenbereich 17 angeordnet ist, wobei die Bildaufnahmeeinrichtung 21 innerhalb einer zylindrischen längs orientierten Kontur angeordnet ist und dennoch eine schräg zu der Längsrichtung orientierte Blickrichtung aufweist. Das zu der Bildaufnahmeeinrichtung 21 gehörige Objektiv 24 weist eine optische Achse mit wenigstens einem Knick auf, so dass sie einen längsorientierten Abschnitt 26 und einen schräg orientierten äußeren Abschnitt N20 hat. Hingegen ist die Beleuchtungseinrichtung 23 (33) längs orientiert und weist einen Abstrahlwinkel auf, der größer ist als der Öffnungswinkel o des Objektivs 24 der Bildaufnahmeeinrichtung 21.

### Bezugszeichen:

- 10: Endoskop
- 11: Schaft
- 12: distales Ende des Schafts 11
- 13: proximales Ende des Schafts 11
- 14: Endoskopkopf
- 15: Anschluss am Endoskopkof
- L: Längsrichtung
- 16: distale Endfläche
- 17: erster Flächenbereich der distalen Endfläche 16
- 18: zweiter Flächenbereich der distalen Endfläche 16
- 19: Trennlinie
- 20: erstes Fenster / Lichteintrittsfenster
- 21: Bildaufnahmeeinrichtung
- 22: zweites Fenster / Lichtaustrittsfenster
- 23: Beleuchtungseinrichtung
- b: Öffnungswinkel der Beleuchtungseinrichtung 23
- N17: Flächennormale des Flächenbereichs 17
- N18: Flächennormale des Flächenbereichs 18
- N20: Flächennormale des ersten Fensters 20
- N22: Flächennormale des zweiten Fensters 22
- P: Schnittpunkt, distal zu der Endfläche 16
- 24: Objektiv
- o: Öffnungswinkel des Objektivs 24
- 25: Bildsensor
- 26: Abschnitt der optischen Achse des Objektivs 24
- 27: LED
- M: Mittelpunkt des ersten Fensters 20
- α, β: Winkel
- 28: Arbeitskanal
- 29, 30: Spülkanal
- 32: drittes Fenster / Lichteintrittsfenster
- 33: dritte Beleuchtungseinrichtung
- 34: Linie
- 35: Parallele
- 36: Parallele
- 37: LED

## Patentansprüche

1. Endoskop (10), insbesondere Wirbelsäulenendoskop,
mit einem länglichen Schaft (11), der sich entlang einer Längsrichtung (L) erstreckt und an dessen distaler Endfläche (16) ein erstes Fenster (20) und ein zweites Fenster (22) angeordnet sind, wobei dem ersten Fenster (20) eine Bildaufnahmeeinrichtung (21) und dem zweiten Fenster (22) eine Beleuchtungseinrichtung (23) zugeordnet ist,
wobei das erste Fenster (20) und das zweite Fenster (22) aufeinander zu geneigt sind.

2. Endoskop nach Anspruch 1, **dadurch gekennzeichnet, dass** das erste Fenster (20) eine ebene Lichteintrittsfläche aufweist.

3. Endoskop nach einem der vorstehenden Ansprüche, **dadurch gekennzeichnet, dass** das zweite Fenster (22) eine ebene Lichtaustrittsfläche aufweist.

4. Endoskop nach einem der vorstehenden Ansprüche, **dadurch gekennzeichnet, dass** die Beleuchtungseinrichtung (23) eine optische Lichtaustrittsachse (N22) aufweist, die parallel zu der Längsrichtung (L) orientiert ist, und dass die Bildaufnahmeeinrichtung (21) eine optische Achse (N20) aufweist, die in einem von Null verschiedenen spitzen Winkel (α) zu der Längsrichtung (L) orientiert ist.

5. Endoskop nach einem der vorstehenden Ansprüche, **dadurch gekennzeichnet, dass** die Bildaufnahmeeinrichtung (21) ein Kameraobjektiv (24) und einen Bildsensor (25) aufweist, die beide in dem distalen Ende (12) des Schafts (11) fest eingebaut sind, und dass die Beleuchtungseinrichtung (23) eine LED-Lichtquelle (27) aufweist, die in dem distalen Ende (12) des Schafts (11) fest eingebaut ist.

6. Endoskop nach Anspruch 5, **dadurch gekennzeichnet, dass** das Kameraobjektiv (24) einen Objektiv-Öffnungswinkel (o) und die Beleuchtungseinrichtung (23) einen Beleuchtungs-Öffnungswinkel (b) aufweist, der größer ist als der Objektiv-Öffnungswinkel (o).

7. Endoskop nach einem der vorstehenden Ansprüche, **dadurch gekennzeichnet, dass** die distale Endfläche (16) einen ersten Flächenabschnitt (17) aufweist, dessen Flächennormale (N17) gegen die Längsrichtung (L) geneigt ist, wobei das erste Fenster (20) vollständig in dem ersten Flächenabschnitt (17) angeordnet ist, und dass die distale Endfläche (16) einen zweiten Flächenabschnitt (18) aufweist, dessen Flächennormale (N18) parallel zu der Längsrichtung (L) orientiert ist, wobei das zweite Fenster (22) vollständig in dem zweiten Flächenabschnitt (18) angeordnet ist.

8. Endoskop nach einem der vorstehenden Ansprüche, **dadurch gekennzeichnet, dass** das Endoskop (10) einen Arbeitskanal (28) aufweist, der sich längs durch den Schaft (11) erstreckt.

9. Endoskop nach den Ansprüchen 7 und 8, **dadurch gekennzeichnet, dass** der Arbeitskanal (28) eine vorzugsweise vollständig von dem zweiten Flächenbereich (18) umschlossenen Mündung aufweist.

10. Endoskop nach einem der vorstehenden Ansprüche, **dadurch gekennzeichnet, dass** in der distalen Endfläche (16) ein drittes Fenster (32) angeordnet ist, dem eine Beleuchtungseinrichtung (33) zugeordnet ist.

11. Endoskop nach den Ansprüchen 9 und 10, **dadurch gekennzeichnet, dass** der zweite und das dritte Fenster (22, 32) an zwei verschiedenen Seiten der Mündung des Arbeitskanals (28) angeordnet sind in unmittelbarer Nachbarschaft zu dem ersten Flächenbereich (17) angeordnet sind.

12. Endoskop nach einem der vorstehenden Ansprüche, **dadurch gekennzeichnet, dass** die Beleuchtungseinrichtung (23) bezüglich der Bildaufnahmeeinrichtung (21) ausschließlich seitlich innerhalb eines Winkels angeordnet ist, der kleiner als 180°, vorzugsweise kleiner als 90° ist.

13. Endoskop nach einem der Ansprüche 8 bis 12, **dadurch gekennzeichnet, dass** in dem Schaft (11) parallel zu dem Arbeitskanal (28) wenigstens ein weiterer Kanal (29) vorgesehen ist, dessen Durchmesser geringer ist als ein innerer Durchmesser des Arbeitskanals (28).

14. Endoskop nach Anspruch 13, **dadurch gekennzeichnet, dass** der Spülkanal (29) zwischen dem ersten Fenster (20) und dem zweiten Fenster (22) angeordnet ist.

15. Endoskop nach Anspruch 13 oder 14, **dadurch gekennzeichnet, dass** der weitere Kanal (29) eine Mündung aufweist, die sowohl in dem ersten Flächenbereich (17) als auch in dem zweiten Flächenbereich (18) liegt.
